# EUROPEAN PATENT SPECIFICATION

(11) **EP 0 435 365 B1**
(45) Date of publication and mention of the grant of the patent: **22.06.1994**
(21) Application number: 90203248.1
(22) Date of filing: 11.12.1990
(51) Int. Cl.: G01N 7/10, G01N 33/20

(54) **Procedure for the measurement of a gas content of molten metal and probe used thereby**
Verfahren zur Messung des Gasgehaltes in geschmolzenem Metall und dabei benutzte Sonde
Procédé pour la mesure d'un contenu de gaz de métal fondu et sonde utilisée pour ce procédé

(30) Priority: 28.12.1989 BE 8901406
(43) Date of publication of application: 03.07.1991
(73) Proprietor: "VLAAMSE INSTELLING VOOR TECHNOLOGISCH ONDERZOEK", afgekort "V.I.T.O.", 2400 Mol (BE)
(72) Inventor: De Schutter, François J.E., B-2440 Geel (BE); Dekeyser, Jacky C., B-3580 Neerpelt (BE); De Burbure de Wesembeek, Stéphane P.M.T., B-1960 Sterrebeek (BE); Luyten, Jan R., B-3046 Vaalbeek (BE)
(74) Representative: Debrabandere, René

(56) References cited:
- EP-A- 0 114 688
- EP-A- 0 342 154
- US-A- 4 331 023
- INDUSTRIAL LABORATORY, vol. 44, no. 11, November 1978, pages 1546-1549, Plenum Publishing Corp.; A.A. ABRAMOV et al.: "Apparatus for measuring hydrogen content in liquid metals"

## Description

The invention relates to a procedure for the measurement of a gas content of molten metal, according to which a probe, which contains a tube which is closed off at its immersion extremity by a porous ceramic membrane that is permeable for the gas to be measured but is impermeable for the molten metal, in which tube a vacuum is sucked, is brought into the molten metal so that the porous membrane is immersed and, after the creation of the vacuum and the immersion, measures the pressure of the gas to be measured in the tube.

The characteristics of a metal are in general influenced by the presence of gases in the molten metal. Gas dispersed in the molten metal can be released during the solidification and cause gas bubbles in the ingots and pigs. During the further treatment of the metal dispersed gas can also cause pores and cracks.

It is therefore usual from time to time to measure the gas content, for example the hydrogen content, of a bath of molten metal. It is known for this purpose to make use of a probe with a porous ceramic membrane through which the gas to be measured diffuses.

With known procedures of this type, which are usually used for the measurement of the hydrogen content, a propellant is pumped in closed circuit over the probe. The propellant will absorb the gas to be measured from the molten metal after which the propellant is analysed. Such procedures are described in the American patents nos. 2 861 450 and 4 624 128 and in the European patent no. 238 054. Because of the necessity of sending a propellant around these procedures are relatively complicated. The apparatus require rather many and long pipes with the danger that the air from the atmosphere penetrates into the pipes and causes false measurement results
In a procedure of the type intended here the use of a propellant is avoided but a vacuum is created in the probe. The gas to be measured diffuses through the porous ceramic membrane into the space where vacuum prevails and it is sufficient to measure the pressure in this space in order to know the gas content. The pressure of the gas always corresponds to the partial pressure of this gas in the molten metal.

A procedure of this type is known from the American patent no. 4 331 023. This patent relates in particular to the measurement of the hydrogen content of a bath of liquid tin on which founded glass is cooled and is formed into sheets. The ceramic membrane has the form of a tube closed off at one extremity which connects with its other extremity to a metal tube which is slideably installed in a cover. After the immersion of the membrane a vacuum is sucked through the whole tube after which the tube is closed off and the pressure in the tube is measured.

With this procedure the content can however not be read quickly after the immersion of the probe since still after this immersion the vacuum must be drawn. Furthermore with the immersion of the ceramic membrane gases can be liberated from this membrane since the membrane is brought up to high temperature rather quickly. These gases can adversely affect the measurement and must first be drawn away.

The purpose of the invention is to remedy these disadvantages and to provide a procedure for the measurement of a gas content of molten metal of the aforementioned type which permits a very accurate and quick measurement.

For this purpose a probe is immersed in the metal of which at least the porous membrane is covered gastightly on the outside by a cover which melts in the molten metal and the vacuum is created inside the cover.

Through the presence of the cover the vacuum need not necessarily be applied after the immersion of the ceramic membrane but this vacuum can equally well be applied prior to the immersion. In the latter case it is even possible to treat the probe beforehand so that possible gases, which might be liberated from the ceramic material or other parts of the probe, during the immersion in the molten metal, are already removed beforehand.

The vacuum for that matter is preferably created in the tube prior to the immersion of the probe into the metal.

It is advantageous to create the vacuum inside the cover by sucking via the tube and therefore through the porous membrane.

In a suitable embodiment of the invention the probe is heated prior to the immersion and the gases thereby liberated inside the tube from the material of the probe are pumped away.

Since the gases are removed prior to the actual measuring these cannot disturb or delay the measuring. During the measuring only the gas to be measured that diffuses from the metal through the porous membrane still enters into the tube.

With this heating the probe is preferably maintained at a temperature higher than 150 degrees Centigrade for at least 15 hours.

The procedure described above is particularly suitable for the measurement of the hydrogen content of a liquid nonferrous metal.

The invention also relates to a probe which is particularly suitable for the application of the procedure according to one of the preceding embodiments, which probe contains a tube which is closed off at its immersion extremity by a ceramic membrane, and which is characterised in that it includes a cover, which is meltable in the molten metal of which the gas content must be measured, which cover gastightly covers the membrane at least on the outside so that inside the cover a space is formed in which the vacuum can be created.

In a particular embodiment of the invention the probe includes a body to which the cover is attached by its open extremity, which body together with the cover forms the aforementioned space in which the vacuum can be created and in which at least the membrane is located.

Suitably the body connects to a tube which surrounds the above mentioned tube closed off by the membrane and on its extremity away from the body, is closed off around the latter tube.

The tube to which the membrane connects is also preferably closed prior to use whereby a vacuum prevails in this tube and therefore also inside the cover and the membrane.

The probe is then ready for use and can be kept for a considerable time in this condition. With the measuring no vacuum must still be created. It is sufficient to connect the tube to which the membrane is connected to a pressure gauge and to immerse the probe in the molten metal.

In a particular embodiment of the invention the membrane has the form of a tube which is closed at one extremity.

A suitable material for the membrane is aluminium oxide.

The tube which is closed off by the membrane is preferably manufactured of metal.

The connection of this metal tube to the ceramic membrane needs to withstand sufficiently long, namely at least five minutes, the high temperature of the metal in which the probe is immersed and also needs to withstand the thermal shock which develops with the immersion.

The metal tube and the ceramic membrane are preferably also welded to each other.

Other details and advantages of the invention will appear from the following description of a procedure for the measurement of a gas content of molten metal and of a probe used thereby, according to the invention.

This description is only given as example and does not restrict the invention.

The reference numbers relate to the drawings attached hereto.
Figure 1 is a view of a device used for the measurement of a gas content of molten metal according to the invention.
Figure 2 shows a cross-section of the probe from the device from figure 1, but drawn to a larger scale.
Figure 3 shows a section of a part from the probe from figure 2 but in relation to another device of the probe.
Figure 4 shows a section of the part from figure 3, but in relation to yet another device of the probe according to the invention.

In the various figures the same reference numbers relate to the same elements.

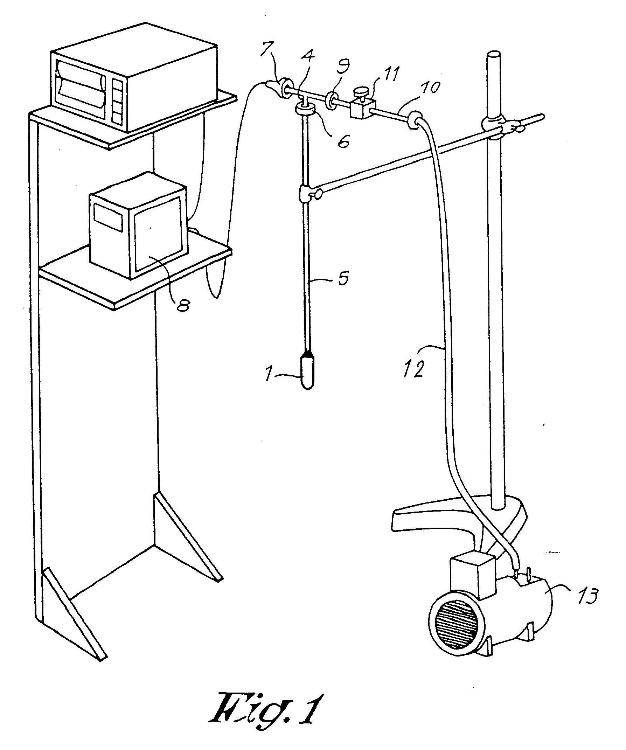

For the measurement of the hydrogen content of molten aluminium according to the invention use is made of a device as shown in figure 1.

This device contains a probe 1 which itself principally consists of a ceramic membrane 2 and an aluminium cover 3 which surrounds the membrane gastightly.

The membrane 2 is attached to the lower extremity of an upright inner tube 4 while the cover 3 is attached to the lower extremity of an outermost tube 5 which surrounds the inner tube 4. A seal 6 gastightly closes off the upper extremity of the tube 5, around the inner tube 4.

One extremity of the T-shaped upper extremity of the inner tube 4 connects to an absolute pressure gauge 7. This pressure gauge 7 is in its turn connected to a reading apparatus 8 and a recording apparatus.

The other extremity of the T-shaped upper extremity of the inner tube 4 connects by means of a coupling 9 to a tube part 10 in which a tap 11 is mounted. This tube part 10 is in turn connected to a vacuum pump 13 by means of a supple pipe 12.

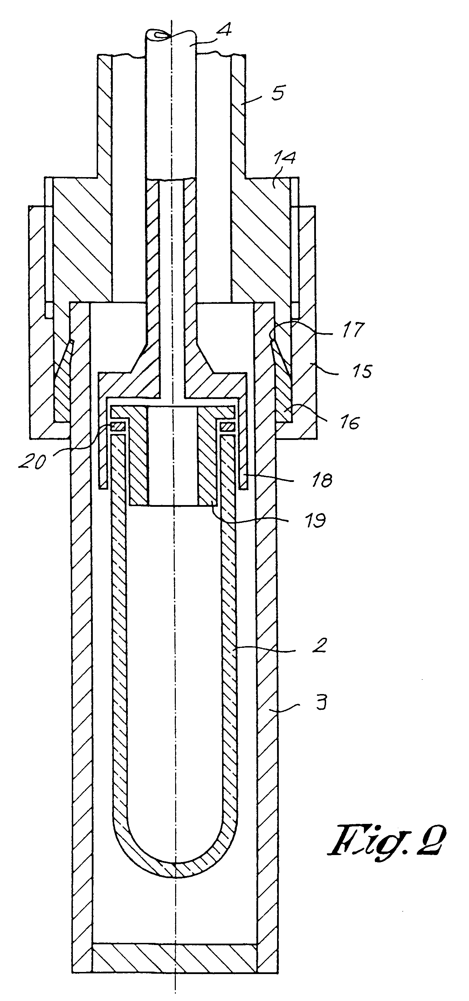

As appears in detail from figure 2, the outer tube 5 is provided underneath with a widened body 14 that is provided externally with a screw thread. The tube 5 and the body 14 form a unit of stainless steel.

A hollow nut 15 is screwed over the body 14. This nut 15 presses a clamping ring 16 against the body 14 and the upper extremity of the cover 3. The extremity is provided with a small groove 17 into which the clamping ring 16 presses.

The cover 3 has the form of a tube which is closed at one extremity. The cover 3 must melt with the immersion of the probe into the molten aluminium and is therefore preferably manufactured of the same metal as the metal of which the gas content has to be measured, and is therefore in this case of aluminium.

The membrane 2 is manufactured of porous ceramic material that allows through hydrogen from the molten aluminium but does not allow the molten aluminium itself through.

The membrane 2 principally consists of aluminium oxide and has an average pore diameter between 10 and 20 micrometer, with a maximum pore diameter of 40 micrometer.

In order on the one hand to obtain this large pore diameter and on the other hand to give sufficient mechanical strength to the membrane 2 this membrane is manufactured starting with a powder with a bimodal particle size distribution. A fraction with relative course particles, which must ensure the desired pore distribution, is mixed with a second fraction of finer particles which are well sinterable and with a binding agent known under the trade name ACRAWAX. Subsequently the powder is pressed isostatically cold using lubricants such as polyvinyl alcohol (PVA) and polyethylene glycol (PEG). The pressed mixture is subsequently sintered for approximately two hours at a temperature of around 1600 degrees Centigrade for example 1650 degrees Centigrade.

The ratio of the two fractions, the size of the largest particles, the pressure employed, the binding agent and the material of the powders are important for the quality of the membrane.

For the manufacture of a membrane with a pore diameter from 5 to 25 micrometer and with an average of 8 micrometer, 68 percent of an aluminium oxide with a grain size between 125 and 250 micrometer, on the market under the brand name ZPS 402, is mixed with 22 percent of a finer aluminium oxide, on the market under the brand name CS 400/M and 10 percent ACRAWAX. One percent PVA and five percent PEG are used as lubricant. The mixture is pressed with a pressure of 1.5 tons per square metre and it is sintered at 1620 degrees Centigrade.

The inner tube 4 is manufactured of stainless steel. The connection of the membrane 2 to this tube 4 must withstand sufficiently long the temperature of the molten aluminium into which the probe will be immersed, this is at least five minutes. This connection must also withstand the thermal shock which develops with the immersion. The different coefficients of expansion of the ceramic material and the stainless steel must also be taken into account. It is for that reason that the membrane 2 is attached to the tube 4 by soldering and with the use of an intermediate ring with approximately the same coefficient of expansion as the ceramic material.

In the embodiment according to figure 2, the inner tube 4 terminates at the bottom end in a small cover 18, into which fits the upper open extremity of the membrane 2. Between the upper extremity of the membrane 2 and the bottom of the small cover 18 a niobium ring 19 is placed. This ring is soldered at the same time to the membrane 2 and to the wall of the small cover 18 by means of a Ti/V/Zr solder 20. The solder is suitably composed of 55% Zr, 19% Ti and 26% V. The soldering is effected in one operation at temperatures between 1200 and 1300 degrees Centigrade under a vacuum of at least 1,33.10⁻³ mbars. The unit is preferably heated for approximately one hour and the materials are maintained for approximately one minute at the soldering temperature after which the materials are cooled off for approximately a half hour.

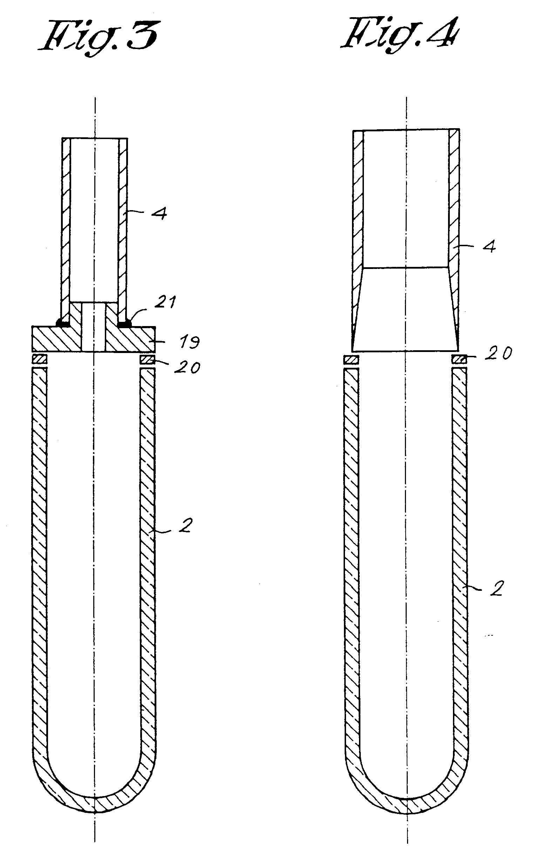

Figures 3 and 4 relate to other embodiments of the connection with solder between the membrane 2 and the inner tube 4. In these figures only this membrane 2, the lower part of the tube 4 and the connection between both parts are shown.

The embodiment according to figure 3 differs from the embodiment from figure 2 because of the fact that the lower part of the inner tube 4 does not form a cover. The niobium ring 19 protrudes with a part into the tube 4 and for the rest is between the tube and the top edge of the membrane 2. The ring of solder 20 with which the niobium ring 19 is soldered onto the membrane 2 as with the embodiment from figure 2 can in this case not provide the connection to the tube 4. The ring 19 is soldered to the tube 4 by a second ring 21 of the soldering material known on the market under the brand name MICROBRASS.

The embodiment according to figure 4 differs from the embodiment according to figure 2 because of the fact that no use is made of a niobium ring. The lower extremity of the tube 4, that terminates in a sharp edge, is only soldered to the membrane 2 by means of the ring of the aforementioned soldering material 20.

With the embodiment according to figures 3 and 4 the soldering is also effected under the same conditions as with the embodiment according to figure 2.

In order to measure the hydrogen content of the molten aluminium, the tap 11 is placed in open position and with the assistance of the vacuum pump 13 suction is created in the inner tube 4 in the membrane 2. The latter is possible because of the fact that the membrane 2 is completely surrounded by the cover 3 and the body 14 and the closed off outer tube 5. Through the membrane 2 vacuum is therefore likewise sucked into the latter named space formed by the latter named elements.

Bringing the probe 1 under vacuum therefore occurs prior to the actual measurement so that the probe is immediately ready for the measurement. It is even possible to bring the probe 1 with possibly the tubes 4 and 5 attached to it under vacuum beforehand prior to mounting the unit in the measurement device as shown in figure 1. The probe 1 can therefore be kept for a long time under vacuum and even be put on the market as such.

It is even recommended to subject the probe 1 to a thermal pretreatment prior to the measuring in order to remove humidity and other gases which could be liberated from the material of the probe 1 during the immersion, in order so to avoid that this humidity or these gases could have an adverse effect on the measurement.

Consequently the complete probe 1 has been brought to a temperature of approximately 200 degrees Centigrade for approximately 24 hours. The gases liberated with that were sucked away with assistance of the vacuum pump 13 and sent by this pump, in a manner not shown in the figure, to an analysis apparatus.

It was furthermore found that especially in the first 15 hours relatively much humidity was liberated. After 24 no more humidity or other gases were liberated. Such gases were also not liberated by afterwards increasing the temperature to 500 degrees Centigrade. It was assumed that heating up to 200 degrees Centigrade for 24 hours is sufficient in order to remove all humidity and gases from the probe.

Obviously the pretreatment of the probe 1 can take place separately from the device shown in figure 1 and therefore prior to mounting the probe 1 in this device. The probe remains dry as long as the vacuum is maintained.

Prior to the measuring the tap 11 is closed and the probe 1 is immersed in the molten aluminium.

The cover 3 melts and the molten aluminium comes into contact with the membrane 2. Hydrogen diffuses from the molten aluminium through the membrane 2 into the space inside the membrane 2 and in the tube 4 a compensating pressure prevails which corresponds to the partial pressure of the hydrogen in the aluminium.

With assistance of the meter 7 the pressure in the inner tube 4 and in the membrane 2 is measured. From this measured pressure the hydrogen concentration of the aluminium can immediately be deduced.

The measured pressure is recorded by means of the recording apparatus.

Other gases than hydrogen are not liberated from the aluminium. Only hydrogen is present in free form in aluminium. Other nonmetallic contamination such as oxygen, nitrogen, carbon monoxide or carbon dioxide are only present in the form of chemical compounds.

In order to prevent that gases which are above the molten aluminium, could enter the tube 4, the whole construction which is immersed must, with the exception of the cover 3 and the membrane 2, be manufactured of a material that dissolves or melts rather slowly in the molten aluminium. It is for that reason that the tubes 4 and 5, the body 14 and the small cover 18 are manufactured of stainless steel while the solders 20 and 21 can also withstand the temperature of the molten aluminium.

The procedure described above allows a very rapid measurement. The vacuum can be created beforehand inside the membrane 2 so that practically immediately after the melting away of the cover 9 the pressure measurement can take place.

The probe 1 can not only be brought under vacuum beforehand but can also be conditioned beforehand, this means that possible gases which could be liberated from the probe itself with the immersion in the molten aluminium, can be liberated, sucked away and possibly be analysed beforehand through a thermal treatment. This also ensures a rapid and accurate measurement.

The thermal shock which occurs with the immersion of the probe 1 in the molten aluminium is considerably alleviated through the presence of the cover 9. While this cover 9 melts away the inside of the probe 1 can heat up slowly.

With the immersion not only the membrane 2 but also a part of the inner tube 4 is immersed and as a result moistened by the molten aluminium. Possible diffusion of gases which are not dispersed in the metal is avoided in this manner.

The invention is in no way restricted to the embodiments described above, and within the scope of the patent application many changes can be applied to the described embodiments, among others with regard to the shape, the composition, the arrangement and the number of the parts which are utilized for the implementation of the invention.

In particular the procedure and the probe are not exclusively limited to the measurement of the hydrogen content in molten aluminium. They are also particularly suitable for the measurement of the hydrogen content in other nonferrous metals.

The cover need not necessarily be manufactured of the same metal as the bath in which the measurement is effected. It must however be manufactured of a metal that melts rather quickly with the immersion in the bath.

The membrane need also not necessarily be manufactured of aluminium oxide. It can also be manufactured of other ceramic materials which can withstand the temperatures of the molten metal.

The probe need also not necessarily have the form of a tube closed off at one extremity.

## Claims

1. Procedure for the measurement of a gas content of molten metals, according to which a probe (1), which contains a tube (1) which is closed off at its immersion extremity by a porous ceramic membrane (2) that is permeable for the gas to be measured but is impermeable for the molten metal, in which (4) tube a vacuum is sucked, is brought into the molten metal, so that the porous membrane (2) is immersed and, after the creation of the vacuum and the immersion, measures the pressure of the gas to be measured in the tube (4) that has diffused through the membrane from the molten metal, characterised in that a probe (1) is immersed in the metal of which at least the porous membrane (2) is covered gastightly on the outside by a cover (3) which melts in the molten metal and the vacuum is created inside the cover (3).

2. Procedure according to the preceding claim, characterised in that the vacuum is created prior to the immersion of the probe (1) in the metal.

3. Procedure according to one of the preceding claims, characterised in that the vacuum is created in the cover (3) by sucking via the tube (4) and through the membrane (2).

4. Procedure according to one of the preceding claims, characterised in that the probe (1) is heated prior to the immersion and the gases thereby liberated in the tube (4) from the material of the probe (1) are pumped away.

5. Procedure according to the preceding claim, characterised in that, prior to the immersion, the probe (1) is heated to a temperature higher than 150 degrees Centigrade for at least 15 hours.

6. Procedure according to the preceding claim, characterised in that the probe (1) is maintained at a temperature of almost 200 degrees Centigrade for almost 24 hours.

7. Procedure according to one of the preceding claims, characterised in that the hydrogen content of a molten nonferrous metal is measured.

8. Probe for the application of the procedure according to one of the preceding claims, which probe (1) contains a tube (4) which is closed off at its immersion extremity by a ceramic membrane (2), characterised in that its includes a cover (3) which is meltable in the molten metal of which the gas content must be measured, which cover (3) gastightly covers the membrane (2) at least on the outside so that inside the cover (3) a space is formed in which the vacuum can be created.

9. Probe according to the preceding claim, characterised in that it includes a body (14) to which the cover (3) is attached by its open extremity, which body (14) together with the cover (3) forms the aforementioned space in which the vacuum can be created and in which at least the membrane (2) is located.

10. Probe according to the preceding claim, characterised in that the body (14) connects to a tube (5) which surrounds the above mentioned tube (4) closed off by the membrane (2) and on its extremity away from the body (14), is closed off around the latter tube (4).

11. Probe according to one of the claim 9 and 10, characterised in that the cover (3) is attached to this body (14) by intervention of a clamping ring (16) by means of a nut (15) which is screwed onto the body (14).

12. Probe according to one of the claims 8 through 11, characterised in that the cover (3) is manufactured of the same metal as the metal for the measurement of the gas content for which the probe is destined.

13. Probe according to one of the claims 8 through 12, characterised in that the membrane (2) has the form of a tube which is closed at one extremity.

14. Probe according to one of the claims 8 through 13, characterised in that the membrane (2) is manufactured of aluminium oxide.

15. Probe according to one of the claims 8 through 14, characterised in that the membrane (2) is connected to the tube (4) by means of solder (20, 21).

16. Probe according to the preceding claim, characterised in that the membrane (2) is connected directly to the tube (4) by means of a solder on the basis of Zr-Ti-v.

17. Probe according to claim 15, characterised in that the membrane (2) is connected to the tube (4) by means of solder (20, 21) by intervention of a metal ring (19) with a coefficient of expansion of the same order as the coefficient of expansion of the ceramic material of the membrane (2).

18. Probe according to the preceding claim, characterised in that the metal ring is a niobium ring.

19. Probe according to one of the claims 17 and 18, characterised in that the tube (4) to which the membrane (2) is attached, terminates in a small cover (18) into which the membrane (2) and the ring (19) fit, and the ring (19) is soldered both to the small cover (18) and to the membrane (2) with a same ring (20) of the solder.

20. Probe according to the preceding claim, characterised in that the solder is manufactured on the basis of Zr-Ti-v.

21. Probe according to one of the claims 17 and 18, characterised in that the metal ring (19) is located entirely between the membrane (2) and the tube (4) and by means of a first solder (20) is soldered to the membrane (2) and by means of another solder (21) is soldered to the tube (4).

22. Probe according to one of the claims 8 and 21, characterised in that the tube (4) which is closed off by the membrane (2) is manufactured of a metal that melts slowly in the molten metal of which the gas content must be measured by the probe.

23. Probe according to the preceding claim, characterised in that the tube (4) is manufactured of stainless steel.

## Patentansprüche

1. Verfahren zur Messung des Gasgehaltes in geschmolzenem Metall, gemäß dessen eine Sonde (1), die ein Rohr (4) umfaßt, das an seinem Eintauchende durch eine poröse Keramikmembran (2) abgeschlossen ist, welche für das zu messende Gas durchlässig ist, jedoch undurchlässig für das geschmolzene Metall, in welchem Rohr (4) ein Vakuum gesaugt wird, in das geschmolzene Metall eingebracht wird, so daß die poröse Membran (2) eingetaucht wird und man, nach der Erzeugung des Vakuums und dem Eintauchen, den Druck des zu messenden Gases, das aus dem geschmolzenen Metall durch die Membran diffundiert ist, im Rohr (4) mißt, dadurch gekennzeichnet, daß eine Sonde (1) in das Metall eingetaucht wird, wovon zumindest die poröse Membran (2) an der Außenseite durch eine Abdeckung (3), die in dem geschmolzenen Metall schmilzt, gasdicht abgedeckt ist und daß das Vakuum in der Abdeckung (3) erzeugt wird.

2. Verfahren gemäß dem vorgenannten Anspruch, dadurch gekennzeichnet, daß das Vakuum vor dem Eintauchen der Sonde (1) in das Metall erzeugt wird.

3. Verfahren gemäß einem der vorgenannten Ansprüche, dadurch gekennzeichnet, daß das Vakuum in der Abdeckung (3) durch Saugen durch das Rohr (4) und durch die Membran (2) erzeugt wird.

4. Verfahren gemäß einem der vorgenannten Ansprüche, dadurch gekennzeichnet, daß die Sonde (1) vor dem Eintauchen erwärmt wird und die dabei im Rohr (4) vom Material der Sonde (1) freigesetzten Gase abgepumpt werden.

5. Verfahren gemäß dem vorgenannten Anspruch, dadurch gekennzeichnet, daß, vor dem Eintauchen, die Sonde (1) für mindestens 15 Stunden auf eine höhere Temperatur als 150 Grad Celsius erwärmt wird.

6. Verfahren gemäß dem vorgenannten Anspruch, dadurch gekennzeichnet, daß die Sonde (1) für etwa 24 Stunden auf einer Temperatur von etwa 200 Grad Celsius gehalten wird.

7. Verfahren gemäß einem der vorgenannten Ansprüche, dadurch gekennzeichnet, daß der Wasserstoffgehalt eines geschmolzenen Nichteisenmetalls gemessen wird.

8. Sonde für die Anwendung des Verfahrens gemäß einem der vorgenannten Ansprüche, welche Sonde (1) ein Rohr (4) umfaßt, das an seinem Eintauchende durch eine Keramikmembran (2) abgeschlossen ist, dadurch gekennzeichnet, daß sie eine Abdeckung (3) umfaßt, die im geschmolzenen Metall, dessen Gasgehalt gemessen werden muß, schmelzbar ist, welche Abdichtung (3) die Membran (2) zumindest an der Außenseite gasdicht abdeckt, so daß in der Abdeckung (3) ein Raum geformt wird, worin das Vakuum erzeugt werden kann.

9. Sonde gemäß dem vorgenannten Anspruch, dadurch gekennzeichnet, daß sie einen Körper (14) umfaßt, an dem die Abdeckung (3) an ihrem offenen Ende befestigt ist, welcher Körper (14) zusammen mit der Abdeckung (3) den vorgenannten Raum bildet, worin das Vakuum erzeugt werden kann und worin zumindest die Membran (2) untergebracht ist.

10. Sonde gemäß dem vorgenannten Anspruch, dadurch gekennzeichnet, daß der Körper (14) an ein Rohr (5) anschließt, das das obenerwähnte Rohr (4) umgibt, das durch die Membran (2) abgeschlossen wird, und an seinem dem Körper (14) abgewandten Ende um letzteres Rohr (4) herum geschlossen ist.

11. Sonde gemäß einem der Ansprüche 9 und 10, dadurch gekennzeichnet, daß die Abdeckung (3) durch das Dazwischentreten eines Klemmrings (16) mittels einer Mutter (15), die an den Körper (14) geschraubt ist, mit diesem Körper (14) verbunden ist.

12. Sonde gemäß einem der Ansprüche 8 bis 11, dadurch gekennzeichnet, daß die Abdeckung (3) aus demselben Metall gefertigt ist wie das Metall für das Messen des Gasgehalts, für das die Sonde bestimmt ist.

13. Sonde gemäß einem der Ansprüche 8 bis 12, dadurch gekennzeichnet, daß die Membran (2) die Form eines Rohrs hat, das an einem Ende geschlossen ist.

14. Sonde gemäß einem der Ansprüche 8 bis 13, dadurch gekennzeichnet, daß die Membran (2) aus Aluminiumoxid gefertigt ist.

15. Sonde gemäß einem der Ansprüche 8 bis 14, dadurch gekennzeichnet, daß die Membran (2) mittels eines Lötmittels (20, 21) mit dem Rohr (4) verbunden ist.

16. Sonde gemäß dem vorgenannten Anspruch, dadurch gekennzeichnet, daß die Membran (2) mittels eines Lötmittels auf der Basis von Zr-Ti-V direkt mit dem Rohr (4) verbunden ist.

17. Sonde gemäß Anspruch 15, dadurch gekennzeichnet, daß die Membran (2) mittels Lötmittel (20, 21) durch das Dazwischentreten eines Metallrings (19) mit einem Ausdehnungskoeffizienten derselben Größenordnung wie der Ausdehnungskoeffizient des Keramikmaterials der Membran (2) mit dem Rohr (4) verbunden ist.

18. Sonde gemäß dem vorgenannten Anspruch, dadurch gekennzeichnet, daß der Metallring ein Niobiumring ist.

19. Sonde gemäß einem der Ansprüche 17 und 18, dadurch gekennzeichnet, daß das Rohr (4), an welchem die Membran (2) befestigt ist, in einer kleinen Abdeckung (18) endigt, in welche die Membrane (2) und der Ring (19) passen, und der Ring (19) mit demselben Ring (20) des Lötmittels sowohl an die kleine Abdeckung (18) als auch an die Membran (2) gelötet ist.

20. Sonde gemaß dem vorgenannten Anspruch, dadurch gekennzeichnet, daß das Lötmittel auf Basis von Zr-Ti-V gefertigt ist.

21. Sonde gemäß einem der Ansprüche 17 und 18, dadurch gekennzeichnet, daß der Metallring (19) völlig zwischen der Membran (2) und dem Rohr (4) angeordnet ist und mittels eines ersten Lötmittels (20) an die Membran (2) und mittels eines zweiten Lötmittels (21) an das Rohr (4) gelötet ist.

22. Sonde gemäß einem der Ansprüche 8 und 21, dadurch gekennzeichnet, daß das Rohr (4), das durch diese Membran (2) abgeschlossen wird, aus einem Metall gefertigt ist, das in dem geschmolzenen Metall, dessen Gasgehalt durch die Sonde gemessen werden muß, langsam schmilzt.

23. Sonde gemäß dem vorgenannten Anspruch, dadurch gekennzeichnet, daß das Rohr (4) aus rostfreiem Stahl gefertigt ist.

## Revendications

1. Procédé pour la mesure de la teneur en gaz de métaux fondus, conformément auquel une sonde (1), qui contient un tube (4) qui est obturé à son extrémité d'immersion par une membrane céramique poreuse (2) gui est perméable au gaz à mesurer, mais qui est imperméable au métal fondu, tube (4) dans lequel on crée du vide par aspiration, est amenée dans le métal fondu de telle sorte que la membrane poreuse (2) soit immergée et, après la création du vide et l'immersion, mesure la pression du gaz à mesurer dans le tube (4) qui a diffusé à travers la membrane à partir du métal fondu, caractérisé en ce qu'une sonde (1) est immergée dans le métal, dont au moins la membrane poreuse (2) est recouverte de manière étanche aux gaz du côté externe par un recouvrement (3) qui fond dans le métal fondu, le vide étant créé à l'intérieur du recouvrement (3).

2. Procédé selon la revendication précédente, caractérisé en ce que le vide est créé avant l'immersion de la sonde (1) dans le métal.

3. Procédé selon l'une quelconque des revendications précédentes, caractérisé en ce que le vide est créé dans le recouvrement (3) par aspiration via le tube (4) et à travers la membrane (2).

4. Procédé selon l'une quelconque des revendications précédentes, caractérisé en ce que la sonde (1) est chauffée avant l'immersion et les gaz ainsi libérés dans le tube (4) sont éliminés de la matière de la sonde (1) par pompage.

5. Procédé selon la revendication précédente, caractérisé en ce que, avant l'immersion, la sonde (1) est chauffée à une température supérieure à 150 degrés centigrades pendant au moins 15 heures.

6. Procédé selon la revendication précédente, caractérisé en ce que la sonde (1) est maintenue à une température d'à peu près 200 degrés centigrades pendant à peu près 24 heures.

7. Procédé selon l'une quelconque des revendications précédentes, caractérisé en ce qu'on mesure la teneur en hydrogène d'un métal non ferreux fondu.

8. Sonde pour l'application du procédé selon l'une quelconque des revendications précédentes, ladite sonde (1) contenant un tube (4) qui est obturé à son extrémité d'immersion par une membrane céramique (2), caractérisée en ce qu'elle englobe un recouvrement (3) qui peut fondre dans le métal fondu dont on doit mesurer la teneur en gaz, ledit recouvrement (3) recouvrant la membrane (2) de manière étanche aux gaz au moins sur le côté externe, si bien qu'à l'intérieur du recouvrement (3), un espace est ménagé, dans lequel le vide peut être créé.

9. Sonde selon la revendication précédente, caractérisée en ce qu'elle englobe un corps (14) auquel est fixé le recouvrement (3) par son extrémité ouverte, ledit corps (14), conjointement avec le recouvrement (3), formant l'espace susmentionné dans lequel le vide peut être créé et dans lequel est située au moins la membrane (2).

10. Sonde selon la revendication précédente, caractérisée en ce que le corps (14) est relié à un tube (5) qui entoure le tube (4) susmentionné obturé par la membrane (2) et, à son extrémité éloignée du corps (14), est obturé autour du tube (4) mentionné en dernier lieu.

11. Sonde selon l'une quelconque des revendications 9 et 10, caractérisée en ce que le recouvrement (3) est fixé à ce corps (14) à l'intervention d'un anneau de serrage (16) au moyen d'un écrou (15) qui est vissé sur le corps (14).

12. Sonde selon l'une quelconque des revendications 8 à 11, caractérisée en ce que le recouvrement (3) est fabriqué avec le même métal que celui dont on doit mesurer la teneur en gaz, la sonde étant destinée à cette mesure.

13. Sonde selon l'une quelconque des revendications 8 à 12, caractérisée en ce que la membrane (2) a la forme d'un tube qui est fermé à une extrémité.

14. Sonde selon l'une quelconque des revendications 8 à 13, caractérisée en ce que la membrane (2) est fabriquée en oxyde d'aluminium.

15. Sonde selon l'une quelconque des revendications 8 à 14, caractérisée en ce que la membrane (2) est reliée au tube (4) au moyen d'un métal d'apport de brasage (20, 21).

16. Sonde selon la revendication précédente, caractérisée en ce que la membrane (2) est reliée directement au tube (4) au moyen d'un métal d'apport de brasage à base de Zr-Ti-V.

17. Sonde selon la revendication 15, caractérisée en ce que la membrane (2) est reliée au tube (4) au moyen d'un métal d'apport de brasage (20, 21) à l'intervention d'un anneau métallique (19) dont le coefficient de dilatation est du même ordre que le coefficient de dilatation de la matière céramique de la membrane (2).

18. Sonde selon la revendication précédente, caractérisée en ce que l'anneau métallique est un anneau en niobium.

19. Sonde selon l'une quelconque des revendications 17 et 18, caractérisée en ce que le tube (4) auquel est fixée la membrane (2) se termine par un petit recouvrement (18) dans lequel la membrane (2) et l'anneau (19) viennent se loger, l'anneau (19) étant brasé à la fois au petit recouvrement (18) et à la membrane (2) avec un même anneau (20) du métal d'apport de brasage.

20. Sonde selon la revendication précédente, caractérisée en ce que le métal d'apport de brasage est réalisé à base de Zr-Ti-V.

21. Sonde selon l'une quelconque des revendications 17 et 18, caractérisée en ce que l'anneau métallique (19) est disposé entièrement entre la membrane (2) et le tube (4) et est brasé au moyen d'un premier métal d'apport de brasage (20), est brasé à la membrane (2) et au moyen d'un autre métal d'apport de brasage (21), est brasé au tube (4).

22. Sonde selon l'une quelconque des revendications 8 et 21, caractérisée en ce que le tube (4), qui est obturé par la membrane (2), est fabriqué en un métal qui fond lentement dans le métal fondu dont la teneur en gaz doit être mesurée par la sonde.

23. Sonde selon la revendication précédente, caractérisée en ce que le tube (4) est fabriqué en acier inoxydable.
